# FASCICULE DE BREVET EUROPEEN

(11) **EP 0 933 108 B1**
(45) Date de publication et mention de la délivrance du brevet: **10.12.2003**
(21) Numéro de dépôt: 99400207.9
(22) Date de dépôt: 29.01.1999
(51) Int. Cl.: B01D 15/08

(54) **Dispositif d'injection discontinue d'un fluide f2 dans une zone z1 ou d'extraction discontinue d'un fluide f1 depuis une zone z1**
Vorrichtung zur diskontinuierlichen Injektion eines Fluides F2 in eine Zone Z1 oder zur diskontinuierlichen Extraktion eines Fluides F1 aus einer Zone Z1
Device for the discontinuous injection of a fluid F2 into a zone Z1 or for the discontinuous extraction of a fluid F1 from a zone Z1

(30) Priorité: 02.02.1998 FR 9801272
(43) Date de publication de la demande: 04.08.1999
(73) Titulaire: Institut Français du Pétrole, 92852 Rueil-Malmaison Cedex (FR)
(72) Inventeur: Capelle, Marianne, 69360 Ternay (FR); Huin, Roland, 69110 Sainte Foy Les Lyon (FR)

(56) Documents cités:
- EP-A- 0 581 153
- WO-A-95/03867
- US-A- 2 985 589

## Description

La présente invention concerne le domaine de la chromatographie et plus particulièrement celui de la chromatographie en lit mobile simulé pour la purification d'un produit donné ou pour la séparation d'un produit contenu dans un mélange.

L'art antérieur est illustré par le brevet EP-A-581 153 qui décrit un filtre comprenant des languettes métalliques flexibles déterminant des fentes pour le passage de liquides qui peuvent être aisément nettoyées.

Dans les procédés de chromatographie on utilise un tamis moléculaire sur lequel les composés du mélange sont adsorbés puis élués à l'aide d'un solvant. En particulier dans le cas de la chromatographie en lit mobile simulé, souvent à contre courant simulé, on associe le plus souvent de larges diamètres de colonnes et de nombreux étages de séparation avec injection ou soutirage périodiques de produits entre deux étages. Le brevet US-A-3214247 décrit par exemple une colonne chromatographique comportant une pluralité d'étages dans laquelle le fluide principal est collecté en totalité en aval d'une grille de collecte positionnée à la sortie d'un premier lit et redistribué en aval d'une grille située à proximité d'un second lit. Le fluide secondaire est introduit par des trous positionnés dans la zone centrale de la zone de collecte où il se mélange directement avec le fluide principal. Ce système présente l'avantage d'avoir de faibles volumes morts et d'induire une perte de charge relativement modérée du fait de sa collecte transversale. Néanmoins, la fonction de mélange n'est pas maîtrisée en totalité et surtout peut induire des phénomènes de rétromélange dans toute la section conique de collecte et/ou de redistribution. Il est de plus certain que lorsque le fluide secondaire est stoppé, le fluide principal peut entrer par les trous dans le moyen utilisé pour introduire ou extraire le fluide secondaire, ce qui vient polluer une fraction dudit fluide secondaire lors de sa réintroduction ou réextraction dans une phase ultérieure.

Le brevet US-A-2985589 décrit un procédé continu de chromatographie d'adsorption employant des lits fixes d'adsorbants et des entrées et des sorties dites mobiles pour les diverses phases formées par la charge, le solvant ou agent de déplacement, le raffinat et l'extrait. Les vannes de contrôles permettent ainsi l'introduction du solvant ou de la charge successivement et périodiquement dans chacun des lits d'adsorption et l'extraction d'un extrait et d'un raffinat à partir de chacun de ces lits. Ces phases d'introduction et d'extraction successives à partir de lits différents mais utilisant la même conduite entraînent une pollution du produit extrait ou introduit dans un lit par le produit extrait ou introduit dans le même lit à la phase précédente. Par ailleurs, dans cette réalisation, le fluide principal peut à tout moment venir polluer dans la conduite le produit que l'on extrait ou que l'on introduit durant la phase au cours de laquelle cette introduction ou cette extraction est stoppée. Ainsi ce système ne permet généralement pas d'obtenir une pureté satisfaisante pour le produit recherché.

Le plus souvent comme cela est par exemple décrit dans la demande de brevet européen au non de la demanderesse EP-A-722355, chaque lit d'adsorbant est séparé du suivant par un système dénommé distributeur-mélangeur-extracteur qui comprend un moyen d'introduction d'un fluide secondaire dans le fluide principal. Ce système tel qu'il est décrit dans cette demande comporte une conduite d'introduction du fluide secondaire dans une chambre d'injection puis dans une chambre de mélange dans laquelle entre également le fluide principal, et en aval de cette chambre de mélange dans le sens de circulation du fluide principal, un moyen de redistribution du mélange formé. Dans cette mise en oeuvre lorsque l'introduction du fluide secondaire est stoppée, seule la pression due à la hauteur de colonne de fluide secondaire s'oppose au reflux du fluide principal dans la conduite d'introduction du fluide secondaire. Ainsi le fluide secondaire reste au contact du fluide principal et la pression dudit fluide secondaire est insuffisante pour s'opposer efficacement à la pollution de ce fluide secondaire par le fluide principal.

La présente invention pallie aux inconvénients précités et vise à éviter ou tout du moins à limiter fortement les risques de pollution des produits introduits ou extraits, en particulier par le fluide principal, durant la phase dans laquelle on n'introduit rien dans la chambre d'injection ou on n'extrait plus rien de la chambre d'extraction. L'invention concerne un dispositif permettant l'injection périodique d'un fluide F2 dans une zone Z1 dans laquelle circule un fluide F1 ou l'extraction périodique du fluide F1 depuis cette zone Z1.

Dans sa forme la plus large la présente invention concerne un dispositif d'injection ou d'extraction comportant au moins un rail d'injection ou d'extraction solidaire d'une chambre d'injection d'un fluide F2 dans une zone Z1 ou d'extraction d'un fluide F1 depuis la zone Z1 dans laquelle ce fluide circule, ledit rail comportant au moins un orifice et de préférence une pluralité d'orifices permettant le passage dudit fluide F2 dans la zone Z1 ou l'extraction dudit fluide F1 depuis la zone Z1, caractérisé en ce qu'il comporte devant chaque orifice au moins un moyen d'obturation au moins partielle dudit orifice, le dit moyen étant situé en aval ou en amont dudit rail dans le sens de circulation dudit fluide F2 et permettant le passage du fluide F2 dans ladite zone Z1 ou l'extraction du fluide F1 depuis ladite zone Z1 dans la chambre d'extraction.

L'un des avantages de ce dispositif est qu'il évite au moins partiellement le mélange de ces deux fluides lorsque l'injection du fluide F2 est stoppée ou l'extraction du fluide F1 est stoppée.

Dans une forme préférée de réalisation de la présente invention, ledit moyen d'obturation au moins partielle de chaque orifice comprend une lame ressort solidaire ou solidarisée sur le rail d'injection comportant une découpe dont la partie située en face de chaque orifice dudit rail d'injection ou d'extraction s'ouvre ou s'entrouvre par action de la pression exercée par le fluide F2 de manière à laisser passer ce fluide F2 dans la zone Z1 ou à laisser passer le fluide F1 depuis la zone Z1 dans la chambre d'extraction. La découpe de cette lame ressort revient le plus souvent en butée sur le rail lorsque l'écoulement du fluide F2 cesse ou lorsque l'extraction du fluide F1 cesse.

Selon une réalisation particulière il est possible d'avoir un moyen d'obturation formé de deux lames ressort coopérant ensemble à la fermeture de l'orifice. Dans la forme préférée de réalisation de l'invention dans laquelle le rail comporte une pluralité d'orifices, on utilisera une lame ressort unique solidarisée sur le rail et comportant un nombre de découpes égal au nombre d'orifices du rail. Une autre forme de réalisation de l'invention s'applique à un dispositif obturant au repos le circuit capable de s'effacer sous l'action d'une certaine pression préalablement connue.

Bien que l'on puisse employer divers matériaux présentant en particulier des caractéristiques d'élasticité choisies en fonction des conditions opératoires, on utilisera le plus souvent une lame ressort en métal élastique. La lame sera par exemple en acier au carbone , en acier inoxydable ou en tout autre métal ayant dans les conditions de travail l'élasticité souhaitée pour remplir sa fonction. Le choix du matériau adéquat par l'homme du métier doit tenir compte des conditions opératoires en particulier du nombre de cycle d'injection ou d'extraction que l'on veut pouvoir assurer, de la pression de travail, de la température et de la composition des divers fluides avec lesquels la lame entre en contact en particulier des risques de corrosion du métal par les fluides employés. Ce choix est un choix relativement facile pour l'homme du métier et largement guidé par les ouvrages de base ou par la description des caractéristiques physiques et des propriétés mécaniques fournies par le vendeur des lames métalliques ressort. L'épaisseur de la lame est habituellement d'environ 0,01 millimètre (mm) à environ 1,5 mm, souvent d'environ 0,02 à environ 1 mm et le plus souvent d'environ 0,05 mm à environ 0,6 mm. On ne sortirait pas du cadre de la présente invention en utilisant un moyen permettant d'imposer une force ou une pression minimale pour obtenir son ouverture. Un tel moyen peut être par exemple une forme particulière de la lame, un formage de lames ou un dispositif élastique annexe permettant de disposer d'une précontrainte qui ne permet l'ouverture que sous un effort minimal connu.

La lame ressort peut être solidarisée au rail par tout moyen bien connu de l'homme du métier. Elle peut être collée, vissée, sérrée, pincée ou soudée. Le plus souvent elle sera vissée ou soudée. Les découpes que comporte cette lame sont en général effectuées avant sa solidarisation avec le rail. Elles ont habituellement une forme adaptée à la fonction qu'elles doivent remplir en liaison avec la forme des orifices.

Il est possible d'avoir des formes de découpes différentes pour une même forme d'orifice. Le plus souvent toutes les découpes d'une même lame ont la même forme de même que tous les orifices d'un même rail. On ne sortirait cependant pas du cadre de la présente invention en utilisant un rail ayant des orifices de formes différentes et/ou une lame ayant des découpes de formes différentes. Le sens de la découpe est habituellement choisi en fonction du sens de l'écoulement du fluide F1. Ce choix résulte de la fonction que remplit cette découpe qui doit s'opposer au passage du fluide F1 dans la chambre d'injection ou d'extraction de fluide lorsque l'injection ou l'extraction de ce fluide est stoppée. Ainsi, bien que cela ne soit pas une obligation absolue, la découpe sera très souvent faite dans le même sens que celui dans lequel le fluide F1 circule.

Dans le cadre de la présente invention, les fluides F1 et F2 peuvent circuler à courant croisé, à co-courant ou à contre courant.

L'invention sera mieux comprise au vu des figures suivantes :
- la figure 1 représente schématiquement en vue éclatée le dispositif de l'invention et les figures 1a et 1b montrent une variante,
- les figures 2a, 2b, 2c et 2d montrent la forme que peut avoir une découpe simple et les figures 2e et 2f montrent la forme que peut avoir une découpe à double lame à ouverture symétrique,
- la figure 3 montre schématiquement une colonne chromatographique comportant le dispositif selon l'invention.

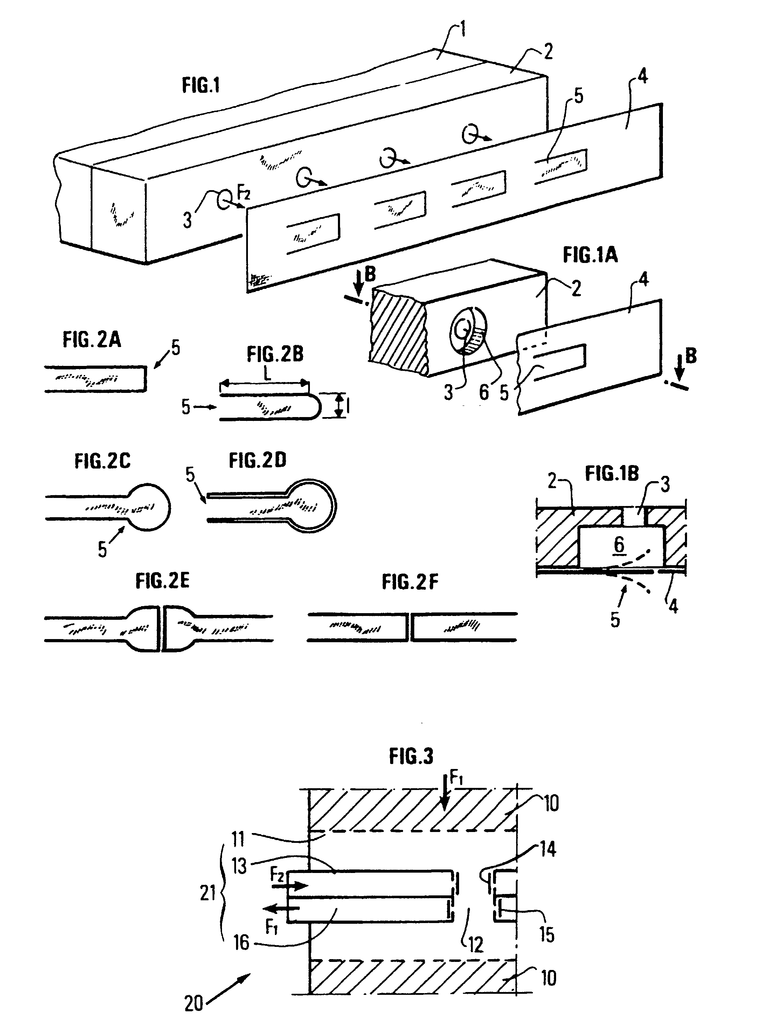

Selon la figure 1, la chambre 1 d'injection ou d'extraction du fluide F2 comporte un rail 2 comportant une pluralité d'orifices 3 sensiblement circulaires et la lame ressort 4 comporte une pluralité de découpes 5 dimensionnées et positionnées de manière à ce que l'extrémité de chaque découpe recouvre entièrement un orifice et soit plaquée sur le rail lorsque la circulation du fluide F2 par exemple est stoppée. Cette schématisation ne doit pas être considérée comme une limitation de la présente invention. Sur la figure 1a est schématisée une autre forme de réalisation de l'invention et cette forme est vue en coupe suivant l'axe BB sur la figure 1b. Les chiffres et les lettres de référence désignent les mêmes élements sur chacune des figures. Sur ces figures 1a et 1b, le rail 2 comporte un orifice 3 formant une chambre 6 de dimension suffisante pour que la découpe 5 dans la lame ressort 4 puisse également s'ouvrir vers l'intérieur de cette chambre.

La figure 3 montre schématiquement l'utilisation du dispositif de la présente invention dans une colonne 20 chromatographique comportant une pluralité d'étages 10 d'adsorption sur un solide (tamis moléculaire par exemple) séparés par des plateaux de distribution 21. Sur cette figure on a schématisé un seul plateau distributeur de fluide comportant deux dispositifs selon l'invention, l'un permettant l'injection d'un fluide F2 de l'extérieur dans la colonne, l'autre permettant l'extraction d'un fluide F1 de la colonne vers l'extérieur. Cette figure ne doit pas être considérée comme limitative. Le fluide F1 traverse le solide adsorbant 10 soutenu par une grille 11 du plateau et parvient dans l'espace 12 dans lequel au cours d'une étape, pendant une durée déterminée, on introduit le fluide F2 par l'intermédiaire de la chambre d'alimentation 13 munie du dispositif 14 selon l'invention schématisé ici par un double trait ou dans une autre étape on extrait pendant une durée déterminée le fluide F1 par l'intermédiaire du dispositif 15 selon l'invention et de la chambre d'extraction 16. Dans la colonne, la composition du fluide F1 varie d'un étage à l'autre et varie en fonction des cycles d'injection du solvant et de la charge, et d'extraction du raffinat et de l'extrait.

Le plus souvent on utilise des chambres distinctes pour l'injection du fluide F2 et pour l'extraction du fluide F1. Cependant, dans une forme particulière de réalisation de la présente invention, on pourra utiliser une seule et même chambre pour l'injection du fluide F2, puis pour l'extraction du fluide F1. Dans ce dernier cas, il est possible d'utiliser un rail 2 comportant un nombre d'orifices 3 plus important, sur lequel est solidarisé de chaque côté une lame 4 comportant en alternance au moins un moyen d'obturation 5 au moins partielle d'un orifice du rail et au moins un orifice disposé en regard d'un autre orifice du rail, ladite lame étant située en aval dudit rail dans le sens de circulation du fluide et permettant le passage du fluide F2 dans la zone Z1 par l'intermédiaire de certains orifices et l'extraction du fluide F1 depuis la zone Z1 par l'intermédiaire d'autres orifices.
Pour ce faire, les orifices des lames amont et aval sont décalés de telle sorte que l'orifice d'une lame coïncide avec le moyen d'obturation de l'autre lame.

Le dispositif de la présente invention trouve une utilisation dans tous les cas de génie chimique nécessitant un contrôle d'une injection ou d'une extraction d'un premier fluide en présence simultanée d'un deuxième fluide différent dudit premier fluide.

La présente invention concerne en particulier l'utilisation du dispositif pour l'obtention d'au moins un isomère du xylène à partir d'une coupe d'hydrocarbures contenant des composés aromatiques à 8 atomes de carbone dont l'isomère du xylène recherché. Cette séparation est effectuée par chromatographie et le plus souvent par chromatographie dite à contre courant simulé.

Les exemples suivant illustrent l'invention sans en limiter la portée.

### Exemple comparatif:

Nous utilisons l'hexane liquide comme fluide d'injection F2 et de l'heptane liquide comme fluide principal F1. Le pression de l'ensemble est de 0,15 Mpa et la température de 20 °C. Une chambre d'injection dispose d' un rail d'injection comprenant huit orifices de 5mm de diamètre. L'hexane, en injection, circule à 10m/s dans chaque orifice. L'heptane circule de façon continue à courant croisé avec l'hexane, à une vitesse de 50cm/s devant les orifices.
L'injection d'hexane dure 5 mm puis est arrétée. En fin d'injection la chambre d'injection est remplie d'hexane.Une sonde de réfractomètre est installée dans la chambre pour mesurer la concentration en heptane en fonction du temps. Celle-ci, nulle en fin d'injection évolue avec le temps jusqu'a une valeur maximale. Par cette mesure, le débit de circulation d'heptane dans la chambre est déduit. Il est de 20 l/h dans cet essai.

### Exemple conforme à l'invention

L'exemple précédent est répété mais une lame ressort en acier inox est soudée sur le rail. Elle a une épaisseur de 0,2 mm et comporte huit découpes de forme semi-oblongue telle que schématisée sur la figure 2b. Chaque découpe est située devant un orifice du rail. La longueur L de la découpe est de 15 mm et sa largeur 1 de 7 mm. La perte de charge introduite par le dispositif est de 0,02 Mpa lors de l'injection d'hexane. Lorsque l'introduction de l'hexane est stoppée, la partie découpée de la lame revient se plaquer sur le rail limitant fortement le passage du fluide principal dans la chambre d'injection. On mesure, en fonction du temps, la concentration en heptane dans la chambre comme précédement à partir du moment où l'hexane est stoppé. Le débit de circulation d'heptane dans la chambre lorsque l'injection est stoppée est évalué à 2 l/h à partir de ces mesures.La chambre d'hexane est donc beaucoup moins polluée par l'heptane que dans le cas précédent.

## Revendications

1. Dispositif d'injection discontinue ou d'extraction discontinue d'un fluide utilisé pour le contrôle de ladite injection ou ladite extraction comportant au moins un rail (2) d'injection ou d'extraction solidaire d'une chambre (1) d'injection d'un fluide F2 dans une zone Z1 oud'extraction d'un fluide F1 depuis la zone Z1 dans laquelle ce fluide circule, ledit rail comportant au moins un orifice (3) permettant le passage dudit fluide F2 dans la zone Z1, ou l'extraction du fluide F1 depuis la zone Z1, **caractérisé en ce qu'**il comporte devant chaque orifice au moins un moyen (4) (5) d'obturation au moins partielle dudit orifice, le dit moyen étant situé en aval ou en amont dudit rail dans le sens de circulation dudit fluide F2 et permettant le passage du fluide F2 dans ladite zone Z1 ou l'extraction du fluide F1 depuis ladite zone Z1 dans la chambre d'extraction.

2. Dispositif selon la revendication 1 dans lequel le moyen d'obturation au moins partielle de chaque orifice du rail comprend une lame ressort solidaire ou solidarisée sur le rail d'injection comportant une découpe dont la partie située en face de chaque orifice dudit rail d'injection ou d'extraction s'ouvre ou s'entrouvre par action de la pression exercée par le fluide F2 de manière à laisser passer ce fluide F2 dans la zone L1 ou par action de la pression exercée par le fluide F1 de manière à laisser passer ce fluide F1 depuis la zone Z1 dans la chambre d'extraction.

3. Dispositif selon la revendication 1 ou 2 dans lequel le rail comporte une pluralité d'orifices et dans lequel le moyen d'obturation au moins partielle de chaque orifice du rail est une lame ressort unique solidarisée sur le rail et comportant un nombre de découpes égal au nombre d'orifices du rail.

4. Dispositif selon l'une des revendications 1 à 3 dans lequel la lame ressort est une lame en métal élastique.

5. Dispositif selon la revendication 4 dans laquelle la lame ressort est une lame en acier au carbone ou en acier inoxydable.

6. Dispositif selon l'une des revendications 1 à 5 dans lequel la lame ressort a une épaisseur d'environ 0,01 millimètre (mm) à environ 1,5 mm.

7. Dispositif selon l'une des revendications 1 à 6, dans lequel le moyen d'obturation comporte au moins un moyen permettant d'imposer une force ou une pression minimale pour obtenir son ouverture.

8. Dispositif selon l'une des revendications 1 à 7 dans lequel le rail comporte des orifices, et dans lequel une lame est solidarisée de chaque côté du rail et comporte en alternance au moins un moyen d'obturation au moins partielle d'un orifice du rail et au moins un orifice disposé en regard d'un autre orifice du rail, ladite lame étant située en aval du rail dans le sens de circulation du fluide et permettant le passage du fluide F2 dans la zone Z1 par l'intermédiaire de certains orifices et l'extraction du fluide F1 depuis la zone Z1 par l'intermédiaire d'autres orifices, les orifices des lames amont et aval étant décalés de telle sorte que l'orifice d'une lame coïncide avec le moyen d'obturation de l'autre lame.

9. Utilisation du dispositif selon l'une des revendications 1 à 8 pour le contrôle d'une injection ou d'une extraction d'un premier fluide en présence simultanée d'un deuxième fluide différent dudit premier fluide.

10. Utilisation du dispositif selon l'une des revendications 1 à 9 pour l'obtention d'au moins un isomère du xylène à partir d'une coupe d'hydrocarbures contenant des composés aromatiques à 8 atomes de carbone dont l'isomère du xylène recherché.

## Claims

1. Device for discontinuous injection or discontinuous extraction of a fluid that is used for the monitoring of said injection or said extraction that comprises at least one injection or extraction rail (2) that is integral with a chamber (1) for injecting a fluid F2 into a zone Z1 or for extracting a fluid F1 from zone Z1 in which this fluid circulates, whereby said rail comprises at least one orifice (3) that makes it possible for said fluid F2 to pass into zone Z1 or for fluid F1 to be extracted from zone Z1, **characterized in that** in front of each orifice, it comprises at least one means (4) (5) for at least partial blocking of said orifice, whereby said means is located downstream or upstream from said rail in the direction of circulation of said fluid F2 and that makes it possible for fluid F2 to pass into said zone Z1 or for fluid F1 to be extracted from said zone Z1 in the extraction chamber.

2. Device according to claim 1, wherein the means for at least partial blocking of each orifice of the rail comprises a spring blade that is integral with or is made integral with the injection rail that comprises a cutout whose part that is located opposite each orifice of said injection or extraction rail opens or half-opens under the action of the pressure that is exerted by fluid F2, in such a way as to allow said fluid F2 to pass into zone Z1 or under the action of the pressure that is exerted by fluid F1, in such a way as to allow this fluid F1 to pass from zone Z1 into the extraction chamber.

3. Device according to claim 1 or 2, wherein the rail comprises a number of orifices and in which the means for at least partial blocking of each orifice of the rail is a single spring blade that is made integral with the rail and that comprises as many cutouts as there are orifices in the rail.

4. Device according to one of claims 1 to 3, wherein the spring blade is an elastic metal blade.

5. Device according to claim 4, wherein the spring blade is a carbon steel blade or stainless steel blade.

6. Device according to one of claims 1 to 5, wherein the spring blade has a thickness of about 0.01 millimeter (mm) to about 1.5 mm.

7. Device according to one of claims 1 to 6, wherein the blocking means comprises at least one means that makes it possible to exert a minimum force or pressure to open it.

8. Device according to one of claims 1 to 7, wherein the rail comprises orifices and wherein a blade is made integral with each side of the rail and alternately comprises at least one means for at least partial blocking of an orifice of the rail and at least one orifice that is placed opposite another orifice of the rail, whereby said blade is located downstream from the rail in the direction of circulation of the fluid and that makes it possible for fluid F2 to pass into zone Z1 by use of certain orifices and for fluid F1 to be extracted from zone Z1 using other orifices, whereby the orifices of the upstream and downstream blades are shifted in such a way that the orifice of one blade coincides with the blocking means of the other blade.

9. Use of the device according to one of claims 1 to 8 for monitoring an injection or an extraction of a first fluid in the simultaneous presence of a second fluid that is different from said first fluid.

10. Use of the device according to one of claims 1 to 9 for obtaining at least one isomer of xylene from a hydrocarbon fraction that contains aromatic compounds with 8 carbon atoms including the desired xylene isomer.

## Patentansprüche

1. Vorrichtung zur diskontinuierlichen injektion oder diskontinuierlichen Extraktion eines Fluids, das für die Regelung dieser Injektion oder dieser Extraktion verwendet wird und wenigstens eine Injektions- oder Extraktionsschiene (2) umfasst, die fest mit einer ammer (1) zur Injektion eines Fluids F2 in eine Zone Z1 oder zur Extraktion eines Fluids aus der Zone Z1, in der dieses Fluid zirkuliert, verbunden ist, wobei die Schiene wenigstens über eine Öffnung (3) verfügt, die den Durchgang dieses Fluids F2 in die Zone Z1 oder die Extraktion des Fluids F1 aus der Zone Z1 ermöglicht, **dadurch gekennzeichnet, dass** sie vor jeder Öffnung wenigstens ein Mittel (4, 5) zum wenigstens teilweisen Verschluss dieser Öffnung umfasst, dieses Mittel in Strömungsrichtung nach oder vor dieser Zone in Zirkulationsrichtung des Fluids F2 gesehen, angeordnet ist und den Durchgang dieses Fluids F2 in diese Zone Z1 oder die Extraktion des Fluids F1 aus dieser Zone Z1 in die Extraktionskammer ermöglicht.

2. Vorrichtung nach Anspruch 1, bei der das Mittel zum wenigstens teilweisen Verschluss jeder Öffnung der Schiene eine Blattfeder umfasst, die ortsfest oder fest verbunden an der Injektionsschiene angebracht ist und einen Ausschnitt bzw. eine Stanzung umfasst, dessen bzw. deren gegenüber jeder Öffnung der Injektions- oder Extraktionsschiene angeordnete Teil sich durch den durch das Fluid F2 ausgeübten Druck derart öffnet oder halb öffnet, dass dieses Fluid F2 in die Zone Z1 durchgehen kann oder durch Wirkung des durch das Fluid F1 derart ausgeübten Drucks, dass dieses Fluid F1 aus der Zone Z1 in die Extraktionskammer übergehen kann.

3. Vorrichtung nach Anspruch 1 oder 2, bei der die Schiene eine Vielzahl von Öffnungen umfasst und bei der das Mittel zum wenigstens teilweisen Schließen jeder Öffnung der Schiene eine einzige Blattfeder ist, die fest an der Schiene sitzt und eine Anzahl von Ausschnitten gleich der Anzahl der Öffnungen der Schiene umfasst.

4. Vorrichtung nach einem der Ansprüche 1 bis 3, bei der die Blattfeder ein Blatt oder eine Zunge aus elastischem Metall ist.

5. Vorrichtung nach Anspruch 4, bei der die Blattfeder ein Blatt aus Kohlenstoffstahl oder rostfreiem Stahl ist.

6. Vorrichtung nach einem der Ansprüche 1 bis 5, bei der die Blattfeder eine Dicke von etwa 0,01 Millimeter (mm) bis etwa 1,5 mm aufweist.

7. Vorrichtung nach einem der Ansprüche 1 bis 6, bei der das Verschlussmittel wenigstens ein Mittel umfasst, das es ermöglicht, eine Kraft oder einen minimalen Druck, um ihr Öffnen zu erreichen, auszuüben.

8. Vorrichtung nach einem der Ansprüche 1 bis 7, bei der die Schiene Öffnungen aufweist und bei der ein Blatt fest mit jeder Seite der Schiene verbunden ist und abwechselnd wenigstens ein Mittel zum wenigstens teilweisen Verschließen einer Öffnung der Schiene aufweist und wenigstens eine Öffnung über einer anderen Öffnung der Schiene angeordnet ist, wobei dieses Blatt hinter der Schiene in Strömungsrichtung des Fluids gesehen, angeordnet ist und den Durchgang des Fluids F2 in die Zone Z1 vermittels gewisser Öffnungen sowie die Extraktion des Fluids F1 aus der Zone Z1 vermittels anderer Öffnungen ermöglicht, wobei die anströmseitigen und abströmseitigen Blätter bzw. Zungen derart versetzt sind, dass die Öffnung eines Blattes oder einer Zunge mit dem Mittel zum Verschließen des anderen Blattes oder der anderen Zunge zusammenfällt bzw. koinzidiert.

9. Verwendung der Vorrichtung nach einem der Ansprüche 1 bis 8 zur Regelung einer Injektion oder einer Extraktion eines ersten Fluids bei gleichzeitiger Anwesenheit eines zweiten sich vom ersten Fluid unterscheidenden Fluids.

10. Verwendung der Vorrichtung nach einem der Ansprüche 1 bis 9 zum Erhalt wenigstens eines Isomers des Xylols ausgehend von einem Kohlenwasserstoffschnitt, der aromatische Verbindungen mit 8 Kohlenstoffatomen, darunter das gesuchte Isomer des Xylols, enthält.
